# EUROPEAN PATENT SPECIFICATION

(11) **EP 2 542 685 B1**
(45) Date of publication and mention of the grant of the patent: **26.03.2014**
(21) Application number: 11706030.1
(22) Date of filing: 02.02.2011
(51) Int. Cl.: C12P 7/64, C12M 1/40, C11C 3/00

(54) **A PROCESS FOR THE ENZYMATIC SYNTHESIS OF FATTY ACID ALKYL ESTERS**
VERFAHREN ZUR ENZYMATISCHEN SYNTHESE VON FETTSÄUREALKYLESTERN
PROCÉDÉ POUR LA SYNTHÈSE ENZYMATIQUE D'ESTERS ALKYLIQUES D'ACIDES GRAS

(30) Priority: 01.03.2010 US 309122 P
(43) Date of publication of application: 09.01.2013
(73) Proprietor: Trans Bio-Diesel Ltd., 20200 Shfaram (IL)
(72) Inventor: BASHEER, Sobhi, 20173 Sakhnine (IL); HAJ, Maisa, 20200 Shfaram (IL); MOHSEN, Usama, Ibleen 30012 (IL); SHEHADEH, Doaa, 16000 Nazareth (IL); HINDAWI, Ahmad, 20200 Shfar-Am (IL); MASOUD, Emad, 30026 Arara (IL)
(74) Representative: Srinivasan, Ravi Chandran
(86) International application number: PCT/IL2011/000121
(87) International publication number: WO 2011/107977

(56) References cited:
- EP-A2- 1 582 594
- WO-A2-2008/084470
- WO-A2-2009/069116
- ANDREW D. MACKENZIE ET AL: "Production of high-oleic acid tallow fractions using lipase-catalyzed direct interesterification, using both batch and continuous processing", ENZYME AND MICROBIAL TECHNOLOGY, vol. 27, 2000, pages 302-311, XP002653352,
- FORESTI ET AL: "Multiple effects of water on solvent-free enzymatic esterifications", ENZYME AND MICROBIAL TECHNOLOGY, STONEHAM, MA, US, vol. 41, no. 1-2, 10 May 2007 (2007-05-10) , pages 62-70, XP022069882, ISSN: 0141-0229, DOI: DOI:10.1016/J.ENZMICTEC.2006.11.023
- XIN CHEN ET AL: "Effect of several factors on soluble lipase-mediated biodiesel preparation in the biphasic aqueous-oil systems", WORLD JOURNAL OF MICROBIOLOGY AND BIOTECHNOLOGY, KLUWER ACADEMIC PUBLISHERS, DO, vol. 24, no. 10, 13 March 2008 (2008-03-13), pages 2097-2102, XP019617136, ISSN: 1573-0972
- SOK-JOONG KIM ET AL: "Lipase Catalyzed Transesterification of Soybean Oil using Ethyl Acetate, an Alternative Acyl Acceptor", BIOTECHNOLOGY AND BIOPROCESS ENGINEERING, vol. 12, 2007, pages 441-445, XP002653353,

## Description

### Field of the Invention

Disclosed is an enzymatic process for the production of fatty acid alkyl esters for use in the biofuels, food and detergent industries. In this process a fatty acid source and an alcohol or alcohol donor are reacted in the presence of enzymes immobilized on a hydrophobic resin, in the presence of an alkaline aqueous buffer or water. The disclosed process can be operated either batchwise or continuously using a continuous stirred-tank or packed-bed column reactors.

### Background of the Invention

Immobilization of enzymes has been described by a vast number of techniques basically aiming at reducing the cost contribution of enzymes in the overall enzymatic process; facilitating recovery of enzymes from the products; and enabling continuous operation of the process.

Immobilization techniques are in general divided according to the following:
1. Physical adsorption of enzymes to solid supports, such as silica and insoluble polymers.
2. Adsorption on ion-exchange resins.
3. Covalent binding of enzymes to a solid support material, such as epoxidated inorganic or polymeric supports.
4. Entrapment of enzymes in a growing polymer.
5. Confinement of enzymes in a membrane reactor or in semi-permeable gels.
6. Cross-linking enzyme crystals (CLECS's) or aggregates (CLEAS's).

All the aforementioned enzyme immobilization procedures are comprised of the following steps:
1. Dissolving the enzyme in an appropriate buffer system with respect to pH, temperature, type of buffer salts and ionic strength.
2. Adding the solid support into the enzyme solution and mixing for some time till enzyme molecules are immobilized on the solid support.
3. Filtering off the solid support which contains the immobilized enzyme.
4. Washing the support with an appropriate buffer to remove loosely bound enzyme molecules and then drying the solid support.

Interfacial enzymes, mostly lipases, have been immobilized following the aforementioned techniques. These offered immobilized enzyme preparations possessing low synthetic activity and/or short operational half-life time. In an attempt to increase the synthetic activity and stability of immobilized lipases and other interfacial enzymes different activation methods have been applied. These methods include:
1. Binding the surface functional groups of enzymes with hydrophobic residues such as fatty acids or polyethylene glycol.
2. Coating the surface of enzymes with surfactants, such as polyol fatty acid esters.
3. Contacting enzymes with hydrophobic supports, typically polypropylene, which have been pretreated with hydrophilic solvents, such as ethanol or iso-propanol.

None of the above mentioned methods yielded satisfactory results with respect to stabilization and cost-effectiveness of immobilized interfacial enzymes, in order to carry out enzymatic reverse conversions at industrial quantities. Also, it has been reported that most enzymes, when immobilized according to the aforementioned procedures, either lose a significant portion of their synthetic activity or they do not exhibit their full activity performance due to certain constraints imposed by the immobilization procedure, or because of the presence of certain enzyme inhibitors in the reaction medium.

Another major drawback of lipases and phospholipases is their low tolerance towards hydrophilic substrates, in particular short-chain alcohols and short-chain fatty acids (below C4). It has been observed in many research studies that short-chain alcohols and short-chain fatty acids, such as methanol and acetic acid, respectively, are responsible for detaching essential water molecules from the quaternary structure of those enzymes, leading to their denaturation and consequently loss of their catalytic activity. This drawback has prohibited the application of lipases for production of commercial quantities of fatty acids methyl esters "biodiesel" using oil triglycerides and methanol as substrates.

An additional drawback of using immobilized lipases for transesterification/ esterification of a fatty acid source with a free alcohol is the accumulation of the formed glycerol and water by-products on the biocatalyst and therefore prohibiting the substrates from free access to the active site of the immobilized enzyme. Such biocatalysts generally lose their catalytic performance after a few cycles when the same batch of biocatalyst is used.

The present inventors have developed special immobilized enzyme preparations, exhibiting good stability over many production cycles, persisting activity. Examples of such enzyme preparations are disclosed, *inter alia*, in WO/2008/084470, WO/2008/139455 and W02009/069116.

Conditions under which the catalytic reaction is carried out, may adversely affect the stability and efficiency of immobilized enzyme preparations. It is important to have enzyme preparations which retain stability and activity under the reaction conditions.

These and other objects of the invention will become apparent as the description proceeds.

### Summary of the Invention

In one embodiment, the invention relates to a process for the transesterification/esterification of a fatty acid source with an alcohol, to form fatty acid alkyl esters, comprising reacting a fatty acid source and an alcohol or an alcohol donor in the presence of an immobilized lipase preparation, wherein the immobilized lipase preparation comprises at least one lipase immobilized on a hydrophobic porous support and the reaction medium contains an aqueous alkaline buffer solution.

The said aqueous alkaline buffer solution may be a mild aqueous alkaline buffer solution. The said aqueous alkaline buffer solution may be contained in the reaction mixture at a quantity of up to 5% wt. of the fatty acid source. The aqueous buffer solution may have a pH from 7 to about 11, for example any one of 7-8.5, 7-9, 7-9.5, 7-10 and 7-11. The pKa of the supplemented mild alkaline reagent comprising of the buffer solution is higher or equal than the pKa of acids comprising the fatty acid source.

In another embodiment the invention relates to a process for the transesterification/esterification of a fatty acid source with an alcohol, to form fatty acid alkyl esters, comprising reacting a fatty acid source and an alcohol in the presence of an immobilized lipase preparation, wherein the immobilized lipase preparation comprises at least one lipase immobilized on a hydrophobic porous support and the reaction medium contains water. The water is in the form of a water solution with a pH of from 3 to 11. The reaction medium may contain the water or water solution at to 5% wt. of the fatty acid source.

In all embodiments and aspects of the invention, the alcohol may be a short-chain alcohol, for example C₁-C₆ alkyl alcohol, more specifically C₁-C₄ alkyl alcohol, particularly methanol or ethanol. Where said alcohol is methanol said resulting fatty acid esters are fatty acid methyl esters (FAME - Biodiesel). The alcohol may also be a medium-chain fatty alcohol (C₆-C₁₀) or long-chain fatty alcohols (C₁₂-C₂₂). The alcohol donor may be a mono-alkyl ester or a di-alkyl carbonate, such as di-methyl carbonate or diethyl carbonate.

In all embodiments and aspects of the invention, said immobilized lipase is capable of catalyzing the esterification of free fatty acids to yield fatty acid alkyl esters and water as by-product, and the transesterification of triglycerides and partial glycerides to yield fatty acid alkyl esters and glycerol as by-product.

In all embodiments and aspects of the invention related to the use of an alkaline buffer or alkaline solution, the amount of said alkaline buffer or solution in the reaction medium is from 0.001 to 5% wt. of the fatty acid source.

In all embodiments and aspects of the invention, said at least one lipase may be a lipase derived from any one *Rhizomucor miehei, Pseudomonas sp., Rhizopus niveus, Mucor javanicus, Rhizopus oryzae, Aspergillus niger, Penicillium camembertii, Alcaligenes sp., Acromobacter sp., Burkholderia sp., Thermomyces lanuginosa, Chromobacterium viscosum, Candida antarctica B, Candida rugosa, Candida antarctica* A, papaya seeds and pancreatin. The lipase preparation may comprise at least two lipases which may be each separately immobilized on a hydrophobic support or co-immobilized on the same hydrophobic support. The said lipases may possess identical or different regio-specificity. The said lipases are capable of simultaneously or consecutively catalyzing the esterification of free fatty acids to yield fatty acid alkyl esters and water as by-product, and the transesterification of triglycerides and partial glycerides to yield fatty acid alkyl esters and glycerol as by-product.

In all embodiments and aspects of the invention, said support may be any one of hydrophobic aliphatic polymer-based support and hydrophobic aromatic polymer-based support. The said hydrophobic polymer support may be comprised of linear or branched organic chains. The said support may comprise macroreticular organic polymer or co-polymer chains. The said support may be porous or non-porous inorganic support, which may be hydrophobic or is coated with hydrophobic organic material. The said organic material may be a linear, branched, or functionalized hydrophobic organic chain.

In all embodiments and aspects of the invention where an alkaline buffer solution is used, said aqueous alkaline buffer solution may be a solution of an inorganic alkaline salt or an organic base. The said alkaline buffer solution may be a solution of any one of an alkaline metal hydroxide, carbonate, bicarbonate, phosphate, sulfate, acetate and citrate, a primary, secondary and tertiary amine, and any mixture thereof. In specific embodiments, the said alkaline buffer solution may be a solution of a weak base selected from sodium or potassium bicarbonates and carbonates. In some specific embodiments of the process of the invention, the said alkaline buffer solution may be added to said fatty acid source in a pre-mixing stage or directly to the reaction medium.

In all embodiments and aspects of the invention where an alkaline buffer solution is used, the content of said alkaline buffer solution in the transesterification/esterification reaction medium may be in the range of 0.001-5% wt. of the oil feedstock., for example 1-2% wt. of the oil feedstock.

In some embodiments of the invention, the fatty acid source may be first mixed with the alkaline buffer solution or with the water or water solution, and the mixture may be then treated with said immobilized lipase preparation, followed by adding said alcohol and allowing the reaction to proceed under suitable conditions until said fatty acid source is converted to fatty acid esters.

In all embodiments and aspects of the invention said fatty acid source may be any one of plant oil, animal fat, algal oil, fish oil, waste oil and any mixtures thereof. The said fatty acid source may comprise free fatty acids, mono-, di- or tri-glycerides, their mixtures at any ratio, in the absence or presence of other minor fatty acid derivatives such as phospholipids and sterol esters. The fatty acid source may be unrefined, refined, bleached, deodorized or any of their combinations.

In all embodiments and aspects of the invention, the reaction may be carried out at a temperature between 10°C and 100°C, specifically between 25-30°C.

In all embodiments and aspects of the invention, the said fatty acid source may be pre-mixed with said alcohol or alcohol donor and with said water or buffer solution in a pre-reaction preparation vessel to form an emulsion which may then be fed together with said immobilized lipase preparation into a transesterification/esterification reaction vessel.

In all embodiments and aspects of the invention, said immobilized lipase may be used in packed-bed column reactors operating in batch or continuous modes.

According to another aspect of the invention there is provided a system for the transesterification/esterification of a fatty acid with an alcohol, to form fatty acid alkyl esters, comprising:
a reaction vessel configured for reacting a reaction medium including a fatty acid and at least one of an alcohol and an alcohol donor in the presence of an immobilized lipase preparation, wherein the immobilized lipase preparation comprises at least one lipase immobilized on a hydrophobic porous support and the reaction medium contains at least one of an aqueous alkaline buffer solution and water.

The system may comprise one or more of the following features, in any desired combination or permutation:
A. The reaction vessel can comprise the immobilized lipase preparation, at least during operation of said system for the production of said fatty acid alkyl esters.
B. Additionally or alternatively to feature A, the reaction vessel can comprise the fatty acid and the at least one of an alcohol and an alcohol donor, at least during operation of said system for the production of said fatty acid alkyl esters.
C. Additionally or alternatively to features A or B, said reaction medium comprises a mixture, said system further comprising a pre-reaction vessel in selective fluid communication with said reaction vessel, said pre-reaction vessel being configured for premixing at least the fatty acid and the at least one of an alcohol and an alcohol donor to form said mixture, and for selectively delivering said mixture to said reaction vessel at least during operation of said system for the production of said fatty acid alkyl esters. The system can optionally further comprise a fatty acid source in selective fluid communication with said pre-reaction vessel and configured for selectively delivering the fatty acid to said pre-reaction vessel at least during said operation of said system, and an alcohol source in selective fluid communication with said pre-reaction vessel and configured for selectively delivering the at least one of an alcohol and an alcohol donor to said pre-reaction vessel at least during said operation of said system. The system can optionally further comprise a buffer source in selective fluid communication with said pre-reaction vessel and configured for selectively delivering the at least one of an aqueous alkaline buffer solution and water to said pre-reaction vessel to be included in said mixture at least during said operation of said system.
D. Additionally or alternatively to features A to C, the system can be configured for selectively delivering one or more of the fatty acid and/or the at least one of an alcohol and an alcohol donor and/or the at least one of an aqueous alkaline buffer solution and water to said pre-reaction vessel each in either a continuous manner or in discrete batches, at least during said operation of said system.
E. Additionally or alternatively to features A to D, the pre-reaction vessel can be configured for selectively delivering said mixture to said reaction vessel in a continuous manner and/or in discrete batches at least during said operation of said system.
F. Additionally or alternatively to features A to E, the system can be configured for selectively and directly delivering to said reaction vessel at least one of the fatty acid; the at least one of an alcohol and an alcohol donor; and the at least one of an aqueous alkaline buffer solution and water.
G. Additionally or alternatively to features A to F, the reaction vessel can comprise a thermal regulation system configured for maintain the reaction medium in said reaction vessel within a selected temperature range.
H. Additionally or alternatively to features A to G, the system can optionally further comprise a retaining arrangement configured for retaining the immobilized lipase preparation within said reaction vessel at least during operation of said system.
I. Additionally or alternatively to features A to H, the system further comprises a product separation vessel in selective fluid communication with said reaction vessel, said system being configured for selectively delivering a reaction mixture including reaction products from said reaction vessel to said product separation vessel, and wherein said product separation vessel is configured for selectively separating a yield of the fatty acid alkyl esters from the reaction mixture delivered thereto. For example, the product separation vessel can be one of a centrifuge and gravity separation system.
J. Additionally or alternatively to features A to I, the reaction vessel is configured for selectively delivering said reaction mixture to said product separation vessel in a continuous manner and/or in discrete batches at least during said operation of said system.
K. Additionally or alternatively to features I to J, the system is configured for selectively delivering said yield of fatty acid alkyl esters from said product separation vessel. For example, the system is configured for selectively delivering said yield of fatty acid alkyl esters from said product separation vessel in a continuous manner and/or in discrete batches.
L. Additionally or alternatively to features A to K, the system is configured for increasing said yield of the fatty acid alkyl esters from the reaction mixture delivered to said product separation vessel. In one configuration of the system having this feature, the system is configured for selectively rerouting said yield of the fatty acid alkyl esters to said reaction vessel to further increase said yield of the fatty acid alkyl esters from the reaction mixture subsequently delivered to said product separation vessel. In another configuration of the system having this feature, the system is configured for selectively rerouting said yield of the fatty acid alkyl esters to an auxiliary reactor module, wherein said auxiliary reactor module comprises an auxiliary reactor vessel and an auxiliary product separation vessel, wherein said further increased yield of the fatty acid alkyl esters is selectively subsequently delivered via said auxiliary product separation vessel.

### Brief Description of the Figures

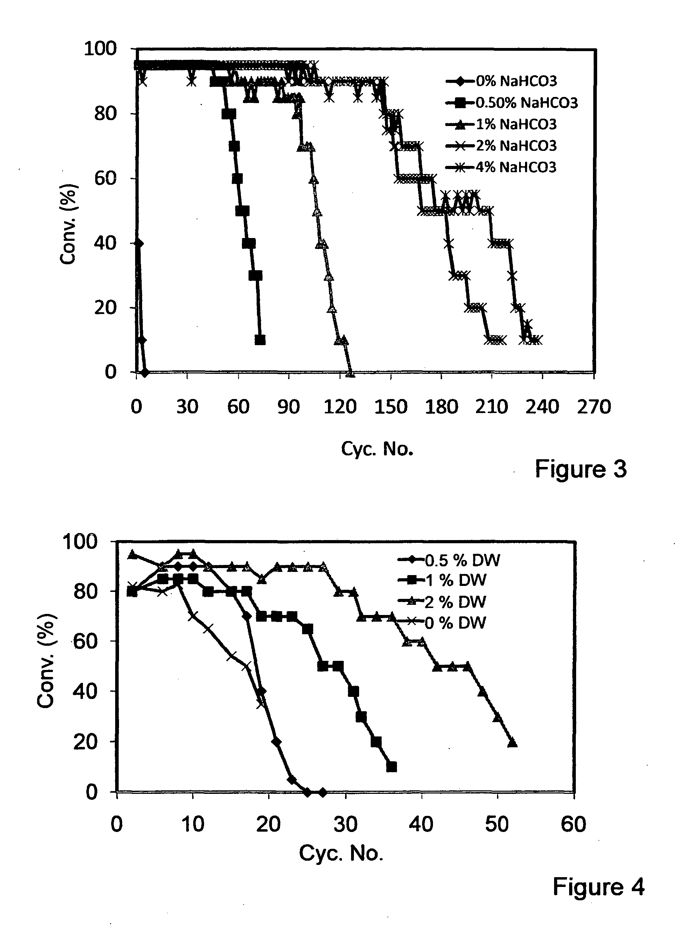

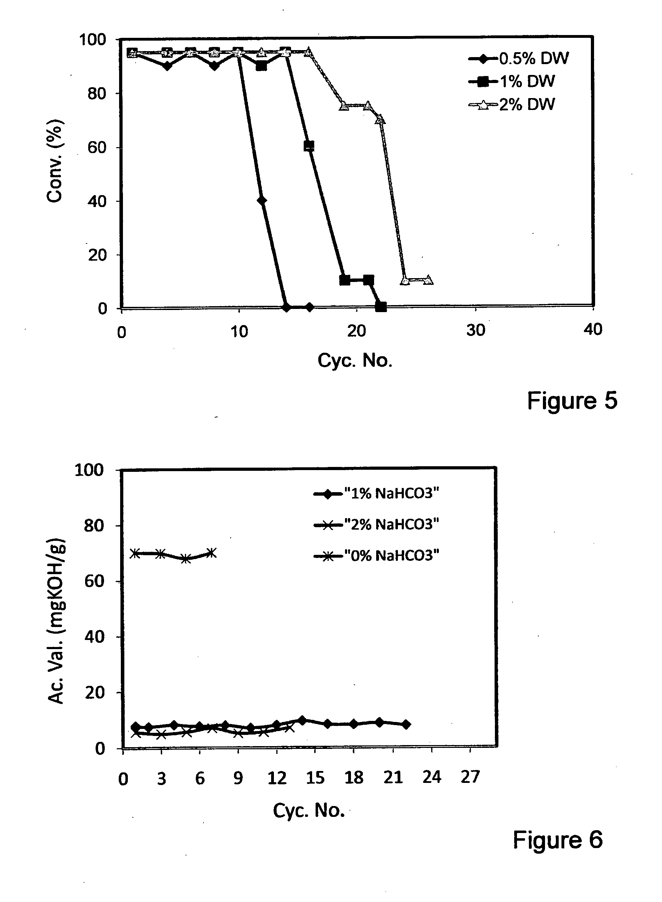

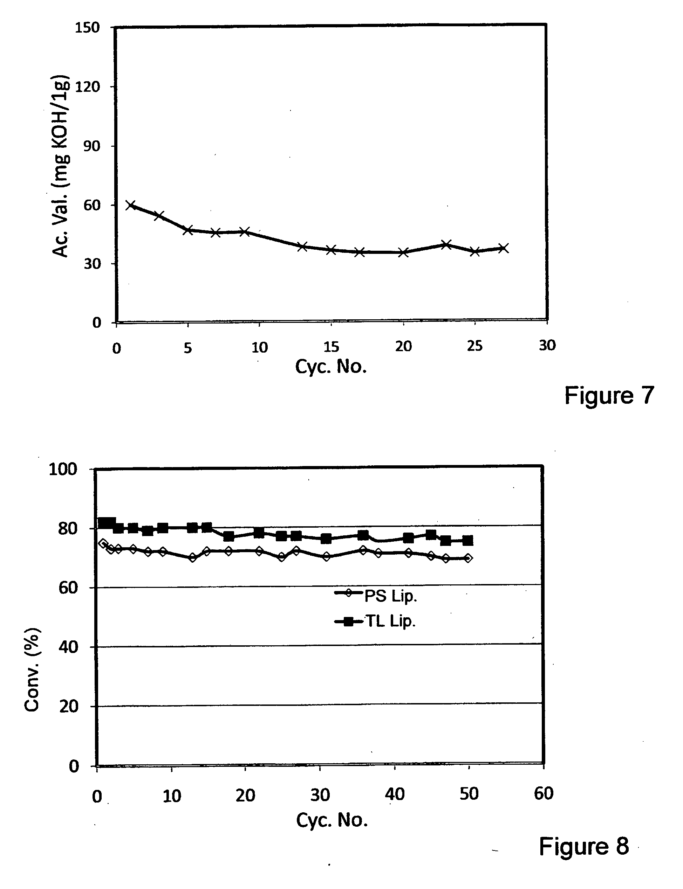

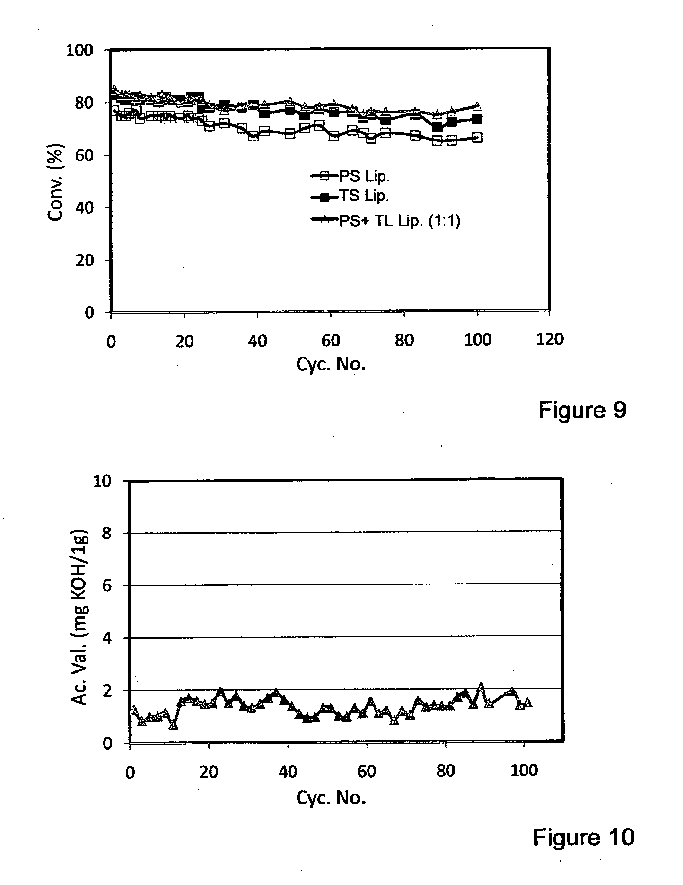

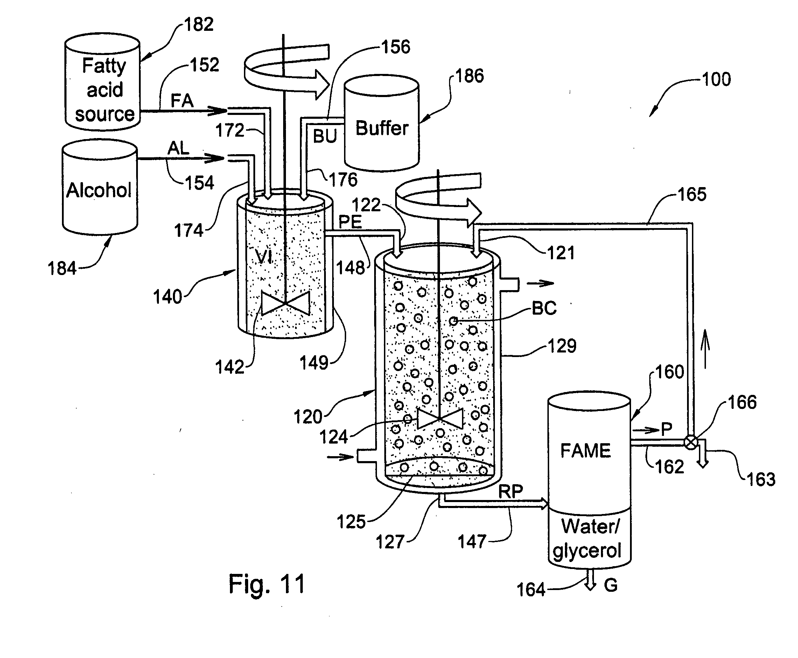

In order to understand the invention and to see how it may be carried out in practice, embodiments will now be described, by way of non-limiting example only, with reference to the accompanying drawings, in which:

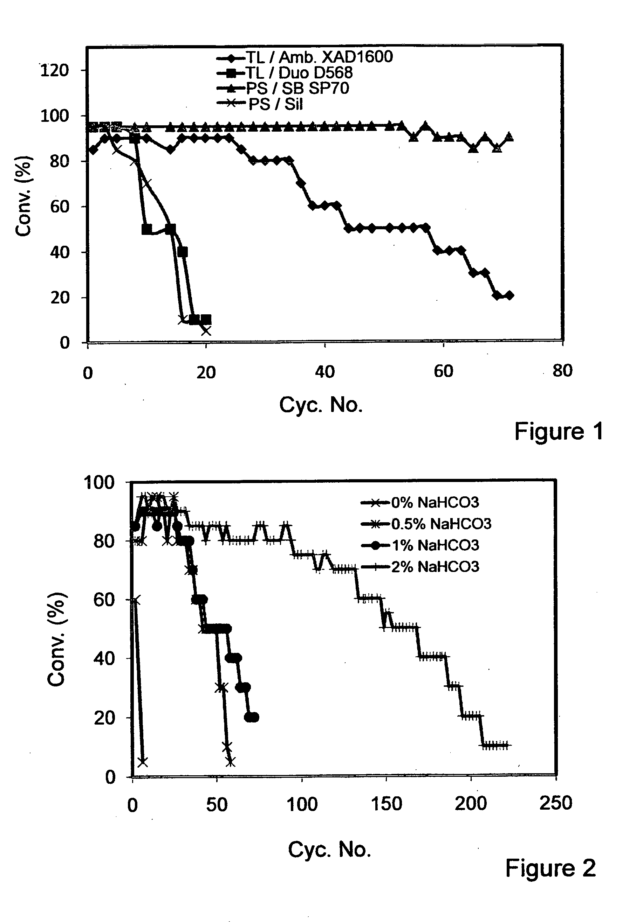

**Figure 1****:** The transesterification activity of lipase *Thermomyces lanuginosa* (TL) immobilized on Amberlite XAD 1600 (Amb. XAD 1600) as a hydrophobic resin and on Duolite D568 (Duo D568) as a hydrophilic resin, and lipase *Pseudomonas sp.* (PS) immobilized on Sepabeads SP70 (SB SP70) as a hydrophobic resin and on porous silica (Sil.) as a hydrophilic resin.
   Abbreviations: Conv. - conversion; Cyc. - Cycle
**Figure 2****:** The conversion of soybean oil to biodiesel and glycerol after 6 hours of reaction at different levels of sodium bicarbonate solution of 0.1M using the same batch of biocatalyst in multiple batch experiments. Biocatalyst was lipase derived from *Thermomyces lanuginosa* immobilized on a hydrophobic and porous polystyrene-divinylbenzene-based resin.
   Abbreviations: Conv. - conversion; Cyc. - cycle
**Figure 3****:** The conversion of soybean oil to biodiesel and glycerol after 6 hours of reaction at different levels of sodium bicarbonate solution of 0.1M using the same batch of biocatalyst in multiple batch experiments. Biocatalyst was lipase derived from *Pseudomonas sp.* immobilized on a hydrophobic and porous polystyrene-divinylbenzene-based resin.
   Abbreviations: Conv. - conversion; Cyc. - cycle
**Figure 4****:** The conversion of soybean oil to biodiesel and glycerol after 6 hours of reaction without water and at different levels of water using the same batch of biocatalyst in multiple batch experiments. Biocatalist was lipase derived from *Thermomyces lanuginosa* immobilized on a hydrophobic and porous polystyrene-divinylbenzene-based resin.
   Abbreviations: Conv. - conversion; Cyc. - cycle; DW - distilled water
**Figure 5****:** The conversion of soybean oil to biodiesel and glycerol after 6 hours of reaction at different levels of water using the same batch of biocatalyst in multiple batch experiments. Biocatalyst was lipase derived from *Pseudomonas sp.* immobilized on a hydrophobic and porous polystyrene-divinylbenzene-based resin.
   Abbreviations: Conv. - conversion; Cyc. - cycle; DW - distilled water
Figure 6: The conversion of a mixture of FFA's and soybean oil to biodiesel, and glycerol and water by-products after 4 hours of esterification/transesterification at different levels of sodium bicarbonate solution of 0.1M using the same batch of biocatalyst in multiple batch experiments. Biocatalyst was lipase derived from *Pseudomonas sp.* immobilized on a hydrophobic and porous polystyrene-divinylbenzene-based resin.
   Abbreviations: Conv. - conversion; Cyc. - cycle; DW - distilled water
**Figure 7****:** The esterification of soybean oil hydrolysate to biodiesel and water after 4 hours of reaction in the presence of 2% sodium bicarbonate solution of 0.1M using the same batch of biocatalyst in multiple batch experiments. Biocatalyst was lipase derived from *Pseudomonas sp.* immobilized on a hydrophobic and porous polystyrene-divinylbenzene-based resin.
   Abbreviations: Ac. Val. - acid value; Cyc. - cycle
**Figure 8****:** The transesterification of fish oil with ethanol after 6 hours of reaction in the presence of 1% wt. of sodium bicarbonate solution of 0.1M using the same batch of biocatalyst in multiple batch experiments. The biocatalysts were lipases derived from *Thermomyces lanuginosa* (TL Lip.) and *Pseudomonas sp.* (PS Lip.) immobilized on Amberlite XAD 1600.
   Abbreviations: Conv. - conversion; Cyc. - cycle
**Figure 9****:** The transesterification of Tallow fat with ethanol after 6 hours of reaction in the presence of 2% wt. of sodium bicarbonate solution of 0.1M using the same batch of biocatalyst in multiple batch experiments. The biocatalysts were *Thermomyces lanuginose, Pseudomonas sp.* lipases (PS Lip.; TL Lip.) immobilized on Amberlite XAD 1600.
   Abbreviations: Conv. - conversion; Cyc. - cycle
**Figure 10****:** The treatment of the transesterification/esterification reaction medium obtained after 4 hours containing FFA value of 7 mg KOH/1g using *Pseudomonas sp.* or *Thermomyces lanuginosa* immobilized on hydrophobic porous resins with *Candida Antarctica* immobilized on a hydrophobic porous resin.
   Abbreviations: Ac. Val. - acid value; Cyc. - cycle
**Fig. 11****:** illustrates schematically a first embodiment of a system for the production of fatty acid alkyl esters according to an aspect of the invention.

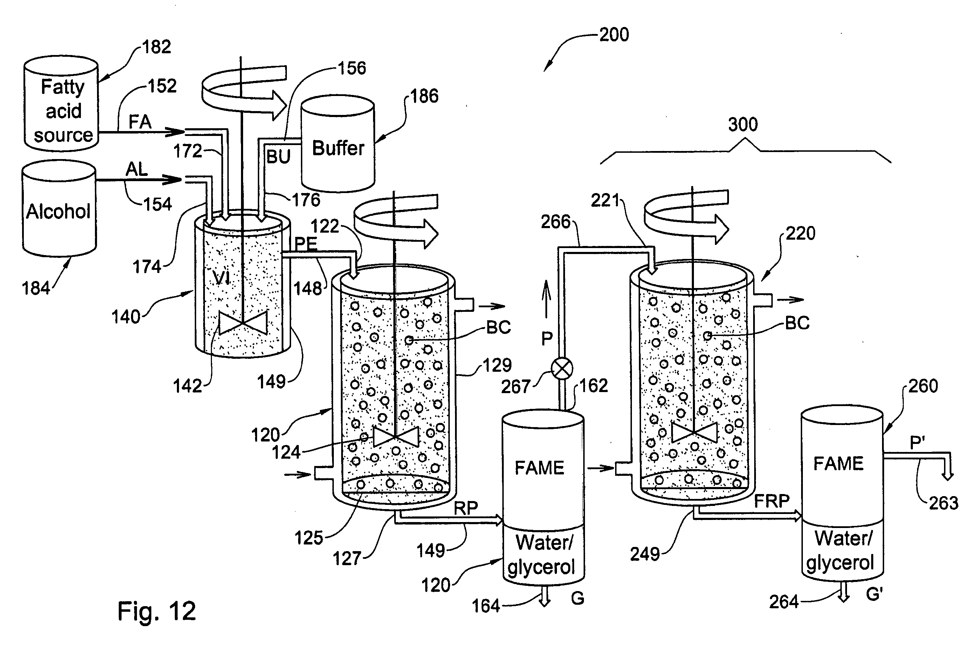

**Fig. 12** illustrates schematically a second embodiment of a system for the production of fatty acid alkyl esters according to an aspect of the invention.

### Detailed Description of the Invention

In search for improvement of enzymatically catalyzed industrial processes, particularly processes for transesterfication/esterification of a fatty acid source with an alcohol in the presence of immobilized lipase/s, the present inventors has developed specific conditions under which the stability of the immobilized lipase/s is preserved over scores of production cycles.

In an embodiment of the invention, the invention relates to a process for the preparation of alkyl esters of fatty acids, specifically short-chain alkyl esters of fatty acids, such as fatty acid methyl and ethyl esters (biodiesel) in a solvent-free alkaline microaqueous system. In specific embodiments, the alkaline microaqueous system is a mild alkaline microaqueous system. The process comprises providing a fatty acid source and reacting it with a free alcohol or an alcohol donor, in the presence of an immobilized lipase preparation, under said alkaline or mild alkaline conditions. Without being bound by theory, pretreatment of the fatty acid source with an alkaline buffer solution would result in neutralizing acids that might have an inhibitory effect on the enzyme. The quantity of alcohol required to complete the reaction up to 100% conversion may be added stepwise or in a one batch. Further, the alcohol may be short-chain alcohol, for example methanol or ethanol. Other alcohol donors may be used in the reaction with the fatty acid source in the presence of a hydrolase and allowing the reaction to proceed under suitable conditions, until said fatty acid source is converted to fatty acid alkyl esters, specifically, fatty acid methyl esters (FAME) or fatty acid ethyl esters, wherein said hydrolase preparation comprises one or more lipases, separately or jointly immobilized on a suitable macroreticular porous hydrophobic polymer-based support.

In an additional embodiment, the transesterification/esterification reaction between the fatty acid source and the alcohol or alcohol donor is carried out in an aqueous microenvrinment, with the addition of water to the reaction mixture. In specific embodiments, water may be added at 0.0001 to 5% wt. of the fatty acid source. By water as used here is meant pure or distilled water, and also "water solutions", which may be, but are not limited to, tap water, sea water or water from any other natural water resource or reservoir, desalinated water, chemically or enzymatically purified or treated water, and any other aqueous solutions. The pH of the reaction system or of the water solution may vary, and may be, for example, about 3-11, for example 4-10, 5-10, 5-9, 6-10, 6-9, or 7-9.

The process of the invention may be carried out while continuously removing the formed glycerol and any excess water from the reaction mixture. The conversion of the fatty acid acyl groups or free fatty acids comprised in said fatty acid source to fatty acid alkyl, specifically methyl esters may be monitored at various time points during the reaction. The reaction medium may be removed by suitable means at any desired time point during the reaction, thereby stopping the reaction, and the formed fatty acid methyl esters and optionally the formed glycerol are isolated from the reaction medium. The reaction may be specifically stopped when the conversion of the fatty acid acyl groups or free fatty acids comprised in said fatty acid source to fatty acid methyl esters has reached at least 70%, for example at least 85%, or at least 90%.

The reaction system may be similar to that described in co-pending WO2009/069116. For example, the production system may use a stirred tank reactor with a bottom sintered glass or stainless steel filter which retains the biocatalyst in the reactor, however allows the reaction medium to permeate through out of the reactor. Such reactor configuration allows by-products, specifically glycerol and water, which are self-desorbed from the immobilized enzyme, to sink to the bottom of the reactor, and permeate out through the filter. The result is continuous removal of the desorbed formed glycerol and also of excess water, out of the reaction medium, leading to shift of the reaction towards synthesis, thereby reaching conversions above 98%. The biocatalyst used in this reactor may be comprised of a single or multi-types of lipases, in consideration of their positional specificity as well as their origin, as described herein. Alternative, two consecutive stirred tank reactors with a bottom filter may be used. A settling tank or centrifuge may be used between the two reactors. The first reactor may contain an immobilized biocatalyst comprised of a single or multi-types of lipases. The role of the settling tank or centrifuge between both reactors is to remove the formed glycerol and excess water from the reaction medium, leading to an increase in the conversion of the raw materials to their corresponding fatty acid alkyl esters to above 98% in the second reactor at reasonable reaction time. Some specific reaction systems and methods are described below.

The terms "reaction mixture", "reaction system" and "reaction medium" may be used herein synonymously.

The use of lipases immobilized on hydrophobic resins in the presence of alkaline buffer solution or water, as in embodiments of the process of the invention, ensures high stability of the enzyme and also avoidance of the accumulation of hydrophilic substances, such as water and the formed glycerol by-prodcut, on the biocatalyst. In specific embodiments of the process of the invention uses 0.001-5% alkaline or mild alkaline buffer solution, for example 0.01-5%, 0.05-5%, 0.1-5%, 0.5-5%, such as 0.001%, 0.01%, 0.05%, 0.1%, 0.5%, 0.75%, 1%, 1.5%, 2%, 2.5%, 3%, 3.5%, 4%, 4.5% or 5%. In specific embodiments of the process of the invention where water is used, the water is used at levels of 0.0001-5% water, for example 0.001-5%, 0.01-5%, 0.05-5%, 0.1-5%, 0.5-5%, such as 0.0001%, 0.001%, 0.01%, 0.05%, 0.1%, 0.5%, 0.75%, 1%, 1.5%, 2%, 2.5%, 3%, 3.5%, 4%, 4.5% or 5%. As mentioned, when alkaline solution is used, it may neutralize acids typically present in the fatty acid source or produce due to side reactions. Continuous active removal of these by-products may even increase the efficiency of the process. The isolated glycerol may be industrially used.

The fatty acid source used in the process of the invention may comprise at least one of soybean oil, canola oil, algae oil, rapeseed oil, olive oil, castor oil, palm oil, sunflower oil, peanut oil, cotton seed oil, Jatropha oil, crude corn oil, fish oil, animal-derived fat, waste cooking oil, brown grease, oil triglycerides derived from inedible plant sources, partial glycerides and free fatty acids derived from those oils or any mixture of at least two thereof, at any desired ratio.

In all processes of the invention, the fatty acid short-chain alkyl esters formed by the reaction are specifically fatty acid methyl, ethyl, *iso*-propyl or butyl esters (biodiesel). Other medium-chain fatty alcohols (C₆-C₁₀) and long-chain fatty alcohols (C₁₂-C₂₂) might also be used in the process of production of this invention. These longer alcohols may be specifically suitable in the production of waxes, for example for cosmetic products.

The lipases may be lipases derived from *Thermomyces lanuginose*, *Rhizomucor miehei, Mucor miehei, Pseudomonas sp., Rhizopus sp., Mucor javanicus, Penicillium roqueforti, Aspergillus niger, Chromobacterium viscosum, Acromobacter sp., Burkholderia sp., Candida antarctica A, Candida antarctica B, Candida rugosa, Alcaligenes sp., Penicillium camembertii,* papaya seeds and pancreatin, but are not limited thereto.

The lipases may be jointly immobilized on a suitable support, specifically a hydrophobic aliphatic polymer-based support or a hydrophobic aromatic polymeric support. Each of said lipases may be immobilized on a suitable support, wherein the supports on which the said lipases are immobilized are identical or different. Lipases employed may be *regio*-specific to their substrate, or random. When more than one lipase is used, the lipases may be immobilized on the same or on different hydrophobic supports. Lipases co-immobilized on the same support can exhibit identical or different substrate selectivities or *regio-*specificities to their substrates.

Lipases may be regio-specific (or site-specific), each used alone or in combination with lipases of same or different site specificity. When referring to positions sn-1, sn-2- or sn-3, these are positions on the glycerol backbone of the various glycerides. Thus, the lipases used in the process of the invention may possess selectivity towards sn-2 position higher than that of random lipases, i.e. their favour catalyzing the reaction between the alcohol or alcohol donor with the fatty acyl group of the sn-2 position, while random lipases exhibit the same transesterification activity for fatty acyl groups at all three positions on the glycerol backbone. Some lipases uniquely exhibit positional activity on sn-2 position, especially under specific conditions determined by the substrates, products, etc. Other lipases used in the process of the invention are sn-1,3 positional specific. They may be used alone or together with a random lipase, specifically lipase that has affinity to partial glycerides, and optionally a third lipase with a high affinity to the sn-2 position.

The support is specifically a porous and macroreticular hydrophobic support, which may be organic or inorganic. Examples of supports are porous inorganic supports, such as, but not limited hydrophobized silica- or and alumina-based supports, and hydrophobic organic supports such as, but not limited to polymeric or polymer-based support. The supports may optionally contain active functional groups selected from epoxy or and aldehyde groups, or ionic groups.

The insoluble support used in the processes of the invention is specifically a porous and reticular hydrophobic aliphatic or aromatic polymer-based support, such as Amberlite^{R} XAD 1600 and Sepabeads^{R} SP70 both comprised of porous microreticular resin prepared from divinylbenzene or from a mixture of divinylbenzene and polystyrene, Amberlite^{R} XAD 7HP comprised of microreticular aliphatic acrylic polymer, and porous aliphatic polymer such as porous polypropylene (Accurel^{R}).

The support may be a reticular hydrophobic polymer comprised of divinylbenzene, or a mixture of divinylbenzene and styrene, and reticular hydrophobic aliphatic polymer comprised of aliphatic acrylic polymers or polyalkene, such as polypropylene. Specific supports are porous matrices, of pore size in the range of 25-1000 Å, and more specifically in the range of 80-200 Å. The support also may be powderous or granular porous hydrophobic silica or other inorganic oxides. The support also may be powderous or granular porous hydrophobicized silica or other inorganic oxides. In specific embodiments, the surface area of the support resins is higher than 100m²/g.

The amount of the alkaline or mild alkaline aqueous solution to be supplemented into the lipase catalyzed transesterification/ esterification reaction between the fatty acid source and the alcohol is generally below 5%wt. of the reaction medium. This alkaline solution is prepared, for example, from an inorganic alkaline base or salt or from an organic base. Inorganic bases and salts are, for example, alkaline metal hydroxides, carbonates, bicarbonates, phosphates, sulfates, acetates and citrates. Organic bases can be, for example, primary, secondary or tertiary amines. Mixtures of these alkaline agents are also contemplated. In the process according to the invention, the pH of the microenvironment of the immobilized enzyme is maintained at alkaline or mild alkaline values. While the addition of distilled water to the reaction system improves the performance of lipases immobilized on hydrophobic support (resins), as illustrated in Figures 4 and 5, the addition of various alkaline buffer, with different pH values depending on the type of base used, resulted in further stabilization of lipases immobilized on hydrophobic supports (resins), as shown, for example, in Figures 2 and 3. Carbonate and bicarbonate buffers are examples of mild bases that are efficient in increasing the stability of lipases immobilized on hydrophobic supports. Other suitable bases are described herein. Generally the pKa of the supplemented alkaline or mild alkaline reagent comprising of the buffer solution is equal or higher than the pKa of acids comprising the fatty acid source. Mild alkaline solution as used herein is generally a solution with a pH of from 7 to about 11, for example, 7-8.5, 7-9, 7-9.5, 7-10 or 7-11. Generally, the amount of alkaline or mild alkaline aqueous solution used is expressed by weight percents (wt.%) on basis of the amount of oil used in the reaction.

The use of lipases immobilized on porous hydrophobic polymer-based supports (resins) in the presence of an alkaline or mild alkaline solution, for example in an amount of 0.01-5% wt., 0.05-5% wt., 0.05-4% wt., 1-5% wt., or 1-4% wt., results in stabilizing the activity of the biocatalysts in the transesterification/ esterification reactions between the fatty acid source and the alcohol. This is shown in the following Examples.

The fatty acid source is at least one of triglycerides, partial glycerides, free fatty acids, phospholipids, esters and amides of fatty acids or a mixture comprised of at least two said sources.

The production of fatty acid alkyl esters is carried out by transesterification or esterification, simultaneously or sequentially. Under such reaction system the biocatalyst activity is maintained with no significant activity losses in multiple uses and also avoids the accumulation of glycerol and water by-products or other hydrophilic compounds on the biocatalyst.

This invention provides processes employing specific immobilized interfacial enzymes that retain high activity and stability over many production cycles. Specifically, lipases and phospholipases preparation are used, in transesterification/esterification reactions. These reactions may be employed in the production of food articles, cosmetics and biofuels ("biodiesel"). Of particular interest, these enzymes may be used for the synthesis of fatty acids short-chain alkyl esters for use as "biodiesel".

The present invention employed stable immobilized interfacial enzymes, of high tolerance towards short-chain alcohols, such as methanol, ethanol and glycerol, as well as short-chain fatty acids, such as acetic acid. The use of these enzyme preparations also prevents accumulation on the immobilized biocatalyst of hydrophilic substances, in particularly glycerol and water.

In an embodiment of the invention there is provided a process for simultaneous or sequential transesterfication/esterification reactions of a fatty acid source with an alcohol using one or more types of lipases, immobilized on a hydrophobic support (resin), in the presence of an alkaline or mild alkaline aqueous solution, for obtaining the desired product, namely, fatty acid alkyl esters, at near to complete conversions during reasonable reaction time, typically below 5 hours. A mild alkaline solution, for example a 0.001M, 0.1M, 0.5M or 1M solution of sodium bicarbonate, may be present in the reaction system in an amount of below about 5% wt. or about 4% wt. of the amount of oil used in the reaction.

As shown in the following Examples, the operational life time of lipases can also be extended by using hydrophobic resin support for lipase immobilization in combination with the use of an alkaline or mild alkaline buffer solution, for example in the range of 0.001-5% wt. in the transesterification/esterification reaction medium. As further shown in the following Examples, the water content of the reaction mixture may be increased regardless of pH value. Thus, in another embodiment, the stability of the biocatalyst increases with increasing the water content of the reaction system by adding water, for example at 0.0001-5% wt. of the fatty acid source, or any of the specific sub-ranges defined above. The results show that the addition of an alkaline solution in the range of 0.0001-5% wt. of the fatty acid source (Figures 2 and 3) or water at 0.001-4% of the fatty acid source (Figures **4** and 5) results in maintaining the enzyme activity and stability over many cycles of the reaction.

The alcohol or alcohol donor employed in the processes of the invention may be a short-chain alkyl alcohol, specifically C₁-C₆ alkyl alcohol, more specifically C₁-C₄ alkyl alcohol, and particularly methanol or ethanol or the alcohol donor may be mono-alkyl ester or dialkyl carbonate, such as dimethyl carbonate. An alcohol donor such as for example dialkyl carbonate can also serve as a source for alkalinity or mild alkalinity of the reaction system.

According to another aspect of the invention there is provided a system for the production of fatty acid alkyl esters. Referring to Fig. **11****,** a first embodiment of such a system, generally designated with the reference numeral **100,** comprises a reactor vessel **120,** a pre-reaction preparation vessel **140,** and a product separation vessel **160.**

Pre-reaction preparation vessel **140** is configured for receiving feedstock materials and buffer (and/or water), for forming a suitable emulsion therefrom, and for feeding the prepared emulsion **PE** (also referred to herein as emulsified feedstock) to the reactor vessel **120.** In particular, such feedback materials may include fatty acid **FA** (for example waste cooking oil) from a fatty acid source **182,** and alcohol **AL** (for example methanol) from alcohol source **184,** and buffer (and/or water) **BU** from buffer/water source **186,** provided via suitable supply lines **152, 154, 156,** respectively, in fluid communication with said pre-reaction preparation vessel **140** via vessel inlets **172, 174, 176,** respectively and suitable valves (not shown).

The pre-reaction preparation vessel **140** defines an internal volume **V1** in which the reaction mixture, including feedstock materials and buffer/water, provided therein via vessel inlets **172, 174, 176,** are mixed together by means of a suitable stirring system **142,** driven by a powered source (not shown), to form emulsion **PE.** The pre-reaction preparation vessel **140** comprises an outer jacket **149** through which a suitable work fluid may be circulated to maintain the volume **V1** at a desired steady state temperature. For example, the work fluid may be oil or water, heated or cooled in a different vessel (not shown) and pumped through the jacket **149** via suitable inlet and exit ports (not shown). In alternative variations of this embodiment, pre-reaction preparation vessel **140** may comprise a system of heating and/or cooling elements, for example electrically powered heating and/or cooling elements, instead of or in addition to the jacket **149.**

Reactor vessel **120** is configured for receiving prepared emulsion **PE** from pre-reaction preparation vessel **140,** for reacting the feedstock materials therein in the presence of a suitable biocatalyst **BC** to produce reaction products **RP,** and for feeding the reaction products **RP** from the reaction mixture to the product separation vessel **160.** Outlet line **148** provides selective fluid communication between pre-reaction preparation vessel **140** and reactor vessel **120** via suitable valves (not shown) and allows the prepared emulsion **PE** prepared by the pre-reaction preparation vessel **140** to be fed to the reactor vessel **120** as desired.

The reaction vessel **120** defines an internal volume **V2** in which the prepared emulsion **PE** in the reaction mixture, provided therein via vessel inlet **122,** is reacted, and the reaction mixture may be stirred by means of a suitable stirring system **124,** driven by a powered source (not shown) to form the reaction products **RP.** The biocatalyst **BC** may comprise a suitable enzyme and is provided in the form of immobilized enzyme beads which remain in the reactor vessel **120** until they become ineffective or are not sufficiently effective, whereupon they may be removed and replaced with new biocatalyst **BC.** For example, the biocatalyst **BC** may comprise lipase derived from *Thermomyces lanuginosa* immobilized on a hydrophobic and porous polystyrene-divinylbenzene-based resin.

The reactor vessel **120** comprises a thermal regulation system in the form of an outer jacket **129** through which a suitable work fluid may be circulated to maintain the volume **V2** at a desired steady state temperature. For example, the work fluid may be oil or water, heated or cooled in a different vessel (not shown) and pumped through the jacket **129** via suitable inlet and exit ports **123.** In alternative variations of this embodiment, the thermal regulation system comprises a system of heating and/or cooling elements, for example electrically powered heating and/or cooling elements, instead of or in addition to the jacket **129.**

The lower part of the reactor vessel **120** comprises an outlet **127,** and a suitable retaining arrangement in the form of filter **125** is provided upstream of the outlet **127** configured for, filtering the reaction mixture, in particular the reaction products **RP** prior to being removed from reactor vessel **120,** and for preventing the biocatalyst **BC** from being removed with the reaction products **RP.**

The product separation vessel **160** is configured for separating out, from the reaction products **RP,** the desired product **P** (fatty acid alkyl ester), from by products including excess water and glycerol **G.** Outlet line **147** provides selective fluid communication between product separation vessel **160** and reactor vessel **120** via suitable valves (not shown) and allows the reaction products **RP** to be fed to the product separation vessel **160_from** the reactor vessel **120** as desired. In this embodiment, the product separation vessel **160** comprises a centrifuge or gravity separation system for carrying out the aforesaid separation, and includes a first outlet **162** for outputting the product **P,** and a second outlet **164** for collecting the excess water and glycerol **G.** Product **P** may be collected via tap **163.**

The system can thus be operated in a continuous production mode, in which prepared emulsion **PE** is fed into the reactor vessel **120,** and the desired product **P** collected in a continuous manner via tap **163.** The emulsion **PE** can be prepared and delivered in a continuous manner to the reactor vessel **120** to top up the volume of reactant therein at the same rate as the reaction products **RP** are being removed from outlet **127.** Alternatively, emulsion **PE** can be prepared and delivered in batches to the reactor vessel **120** to top up the volume of reactant in the reaction mixture at discrete intervals whenever the level of reactants in the reactor vessel **120** drops to a particular minimum level following the continuous removal of reaction products **RP** via outlet **127.** Of course, it is also possible to operate the system **100** to provide the desired product P in batches rather than continuously.

Alternatively, the system **100** may be operated in enhanced yield mode, wherein product **P** is, instead of being immediate collected via tap **163,** rerouted to the reactor vessel **120** via an optional rerouting system, including line **165,** vessel inlet **121** and valve **166,** wherein valve **166** may be selectively operated to divert the product **P** from tap **163.** When rerouted to reactor vessel **120,** the product **P** may be further reacted therein with alcohol **AL,** provided via a separate line (not shown) from source **184,** from a different alcohol source (not shown), or from source **184** via pre-reaction preparation vessel **140,** to produce a higher yield of product **P,** which again may be separated out from byproducts using product separation vessel **160.** When the alcohol is provided via preparation vessel **140,** the latter is first emptied of the prepared emulsion **PE,** and suitable valves prevent fatty acids **FA** and optionally buffer/water being provided by respective **sourcses182** and **186.** Suitable pumps or gravity feeds and controllable valves may be provided for selectively transporting the respective materials through the respective lines **152, 154, 156, 148, 147, 165,** and a suitable controller (not shown) monitors and controls operation of the system.

In at least some alternative variations of the first embodiment, the pre-reaction preparation vessel **140** may be integral with the reactor vessel **120.** For example, the respective internal volumes **V1** and **V2** may be separated by a wall having an opening arrangement corresponding to the line **148.** Alternatively, the respective internal volumes **V1** and **V2** may be contiguous, but internal volume **V1** is sufficiently spaced from the biocatalyst **BC** to provide sufficient time for the emulsion **PE** to form before reaching the biocatalyst **BC.**

In alternative variations of the first embodiment, one, two or all of the fatty acid **FA,** alcohol **AL,** and buffer/water **BU** may be provided directly to the reactor vessel **120,** bypassing the pre-reaction preparation vessel **140.** For example, one or more of the fatty acid source **182,** alcohol source **184,** and buffer/water source **186,** may be in selective fluid communication directly with reactor vessel **120** via suitable supply lines (not shown) bypassing the pre-reaction preparation vessel **140.**

It is appreciated that all components of the system **100** according to the first embodiment, or alternative variations thereof, are of a suitable form and made from suitable materials as known in the art, such as to enable each component to carrying out the respective functions at the respective conditions, including temperature, pressure, pH and so on.

Referring to Fig. **12****,** a second embodiment of the system, designated with the reference number **200,** comprises all the elements and features of the first embodiment, including alternative variations thereof, including all like-numbered components as in Fig. **11****,** *mutatis mutandis,* with some differences. For example system **200** also comprises: a reactor vessel **120,** a pre-reaction preparation vessel **140,** a product separation vessel **160,** fatty acid source **182,** alcohol source **184,** buffer/water source **186,** supply lines **152, 154, 156,** vessel inlets **172, 174, 176,** stirring system **142,** outer jacket **149,** outlet line **148** vessel inlet **122,** stirring system **124,** biocatalyst **BC** outer jacket **129,** inlet and exit ports **123,** outlet **127,** filter **125,** outlet line **147** first outlet **162** second outlet **164;** as disclosed for the first embodiment, *mutatis mutandis.*

However, in the second embodiment, the line **165,** tap **163** and valve **166** of the first embodiment are omitted, and instead an auxiliary reactor module **300** is operatively connected to the first outlet **162** of the product separation vessel **160.**

Auxiliary reactor module **300** comprises an auxiliary reactor vessel **220** and an auxiliary product separation vessel **260,** which in this embodiment are respectively substantially similar to reactor vessel **120** and product separation vessel **160,** *mutatis mutandis.* In operation, the desired product **P** from product separation vessel **160** is routed to the auxiliary reactor vessel **220** via line **266,** valve **267** and vessel inlet **221.** When routed to auxiliary reactor vessel **220,** the product **P** may be further reacted therein with alcohol **AL,** provided via a separate line (not shown) from source **184** or from a different alcohol source (not shown), to produce further reacted products **FRP.** Line **249** enables the further reacted products **FRP** to be transported to the auxiliary product separation vessel **260,** which then operates to separate a higher yield of product P' from byproducts.

System **200** may be operated in a similar manner to system **100,** *mutatis mutandis.*

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention, will be limited only by the appended claims.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following Examples are representative of techniques employed by the inventors in carrying out aspects of the present invention.

### Examples

### General

All experiments were carried out either in glass tubes of 30ml in volume bottomed with a centered glass filter or in mechanically stirred reactors of 500 ml in volume bottomed with a sintered glass filter of porosity of 150-250 µm. Typical reaction medium contained fatty acid source, alcohol, normally, methanol or ethanol in molar basis 1:1 in relation to the fatty acid regardless free or bound on a glycerol backbone (for free fatty acids and monoglycerides 1:1, for diglycerides 1:2, and for triglycerides 1:3 in favor of the alcohol). The fatty acid source was premixed with different amounts of alkaline buffer, in specific embodiments sodium bicarbonate. The reactions were initiated by the addition of lipase immobilized on a hydrophobic resin (10-15%wt.) and the reaction medium was either shaken mechanically or stirred at 30°C. The alcohol amount was added equally in three steps each one hour apart, unless indicated differently. Reaction conversions were followed by taking samples from the reaction medium at different time intervals and analyzing fatty acid components. The conversion to biodiesel was calculated as: 100* peak area of fatty acid alkyl ester/sum of all peaks areas.

Lipase immobilization: Lipases were immobilized following standard procedures where lipase derived from a certain microorganism is solubilized in buffer solution of 0.1M at a certain pH value, for example 7.5. An organic or inorganic polymer resin was introduced into the lipase solution. The mixture was shaken at room temperature for 8 hour. Cold acetone was optionally added into the mixture in order to increase the protein enzyme precipitation on the resin. The mixture was filtered and the enzyme beads were dried to reduce the water content to less than 5%.

Different resins were used including hydrophobic polymer resins based on polystyrene/divinylbenzen, paraffin or any of their combinations, to obtain resins of hydrophobic characteristics. Typical hydrophobic resins used included Amberlite^{R} XAD 1600 (Rohm & Haas, USA) and Sepabeads^{R} SP70 (Resindion, Italy). Typical hydrophilic resins used included Duolite^{R} D568 (Rohm & Haas) and porous silica gel. Lipases may be immobilized separately on a resin or different lipases are co-immobilized on the same resin.

### Example 1

The transesterification activity of lipase derived from *Thermomyces lanuginosa* immobilized on Amberlite^{R} XAD 1600 as a hydrophobic resin and on Duolite^{R} D568 as a hydrophilic resin, and lipase derived from *Pseudomonas sp.* immobilized on Sepabeads^{R} SP70 as a hydrophobic resin and on porous silica as a hydrophilic resin.

*Reaction conditions:* Refined and bleached soybean oil (20g) containing 1% wt. of sodium bicarbonate solution of 0.1M. Methanol (2.5ml) was added stepwise in three equivalent batches each one hour apart. The reaction medium containing 10% wt. lipase preparation was shaken at 300rpm and 30°C. Results are shown in Figure 1.

The results presented in Figure 1 show that both the *Thermomyces lanuginosa* and *Pseudomonas sp.* lipases immobilized on different resins in the presence of 1% wt. of sodium bicarbonate solution showed high transesterification activity during the first 5 cycles using the same batch of enzyme. It was observed that after the 5^{th} batch, when the same batch of enzyme was used, the filtration of the reaction medium from the system became difficult due to the formation of gel-like deposit around the beads of both lipases immobilized on hydrophilic resins, namely Duolite^{R} D568 and porous silica. The interesterification activity of both lipases immobilized on hydrophilic resins decreased sharply in further consecutive batches, and they became inactive after the 10^{th} cycle. In contrast, *Pseudomonas sp.* lipase immobilized on the hydrophobic resin, Sepabeads^{R} SP70, retained more than 80% of its initial activity after 70 cycles, while *Thermomyces lanuginose* lipase immobilized on the hydrophobic resin, Amberlite^{R} XAD1600, retained more than 20% of its initial activity after more than 70 cycles.

### Example 2

### A. The conversion of soybean oil to biodiesel and glycerol after 6 hours of reaction using the same batch of biocatalyst in multiple batch experiments.

*Reaction conditions:* Refined and bleached soybean oil (20g) containing different concentrations of sodium bicarbonate solution of 0.1M. Methanol (2.5ml) was added stepwise in three equivalent batches each one hour apart. Lipase derived from *Thermomyces lanuginosa* immobilized on a hydrophobic and porous polystyrene-divinylbenzene-based resin, was used (10%wt.). The reaction medium was shaken at 300 rpm and 30°C. Results are shown in Figure 2.

### B. The conversion of soybean oil to biodiesel and glycerol after 6 hours of reaction using the same batch of biocatalyst in multiple batch experiments.

*Reaction conditions:* Refined and bleached soybean oil (20g) containing different concentrations of sodium bicarbonate solution of 0.1M. Methanol (2.5ml) was added stepwise in three equivalent batches each one hour apart. Lipase derived from *Pseudomonas sp.* immobilized on a hydrophobic and porous polystyrene-divinylbenzene-based resin, was used (10%wt.). The reaction medium was shaken at 300rpm and 30°C. Results are shown in Figure 3.

Figures 2 and 3 show that the amount of sodium carbonate in the reaction medium has a major role on the operational life of *Thermomyces lanuginosa* and *Pseudomonas sp.* lipases immobilized on hydrophobic resins. It can be seen in Figures 2 and 3 that in the absence of an alkaline solution both immobilized lipases drastically lose their activity after a few cycles, while the same immobilized lipases maintain their transesterification activity over multiple uses in the presence of sodium bicarbonate solution as a base in the reaction system. The results for both immobilized enzymes show that increasing the amount of sodium bicarbonate solution in the reaction medium in the range of 0 - 4% wt. results in decreasing the loss of enzyme activity in multiple uses of the same batch of immobilized enzyme.

### Example 3

### A. The conversion of soybean oil to biodiesel and glycerol after 6 hours of reaction using the same batch of biocatalyst in multiple batch experiments.

*Reaction conditions:* Refined and bleached soybean oil (20g) containing different concentrations of distilled water. Methanol (2.5ml) was added stepwise in three equivalent batches each one hour apart. Lipase derived from *Thermomyces lanuginosa* immobilized on a hydrophobic and porous polystyrene-divinylbenzene-based resin, was used (10%wt.). The reaction medium was shaken at 300rpm and 30°C. Results are shown in Figure 4.

### B. The conversion of soybean oil to biodiesel and glycerol after 6 hours of reaction using the same batch of biocatalyst in multiple batch experiments.

*Reaction conditions:* Refined and bleached soybean oil (20g) containing different concentrations of distilled water. Methanol (2.5ml) was added stepwise in three equivalent batches each one hour apart. Lipase derived from *Pseudomonas sp.* immobilized on a hydrophobic and porous polystyrene-divinylbenzene-based resin, was used (10%wt.). The reaction medium was shaken at 300rpm and 30°C. Results are shown in Figure 5.

Figures 4 and 5 show that the transesterification activity using the same batch of lipases *Thermomyces lanuginosa* and *Pseudomonas sp.* immobilized on hydrophobic resins in multiple experiments is also affected by the amount of water in the reaction system. It can be seen that increasing the water amount from none (zero) to 4% wt. resulted in maintaining higher residual transesterification activity of biocatalyst when used in consecutive cycles. The results presented in Figures 2 to 5 evidently show that using mild base, such as sodium bicarbonate solution in the transesterification reactions is favored for maintaining the activity of lipases immobilized on hydrophobic resins when used in consecutive cycles.

### Example 4

The conversion of a mixture of free fatty acids (FFA's) and soybean oil to biodiesel, and glycerol and water by-products after 4 hours of esterification/transesterification using the same batch of biocatalyst in multiple batch experiments.

*Reaction conditions:* A mixture of free fatty acids soybean hydrolysate (50%wt.) and soybean oil (50%wt.) of initial FFA value 72 mg KOH/1g containing different amount of sodium bicarbonate solution of 0.1M. Methanol (4.5ml) was added stepwise in three equivalent batches each one hour apart. Lipase derived from *Pseudomonas sp.* immobilized on a hydrophobic and porous polystyrene-divinylbenzene-based resin, was used (20%wt.). The reaction medium was shaken at 300rpm and 30°C. Results are shown in Figure 6.

Figure 6 shows that different amount of base solution has a major effect on the simultaneous esterification reaction of FFA present in the reaction mixture comprised of equivalent proportions of soybean oil hydrolysate and soybean oil triglycerides. It can be seen that *Pseudomonas sp.* lipase immobilized on a hydrophobic resin lost its esterification activity when no alkaline solution was added into the esterification/transesterification reaction system, while the same biocatalyst has maintained its activity in consecutive cycles when 1 and 2% wt. of sodium bicarbonate solutions of 0.1 M were added separately into the reaction systems. The results presented in Figure 6 show that the use of *Pseudomonas sp.* lipase immobilized on a hydrophobic resin reduced the FFA content in the presence of 1% and 2%wt. of sodium bicarbonate solution of 0.1M from initial value of 72 mg KOH/1g down to 8 and 6 mg KOH/1g in average, respectively, and maintained this activity in 22 subsequent cycles.

### Example 5

The esterification of soybean oil hydrolysate to biodiesel and water after 4 hours of reaction using the same batch of biocatalyst in multiple batch experiments.

*Reaction conditions:* Free fatty acids soybean hydrolysate (20g) of FFA value of 150 mg KOH/1g containing 1% wt. sodium bicarbonate solution of 0.1M. Methanol (2ml) was added into the reaction medium in one batch. Lipase derived from *Pseudomonas sp.* immobilized on a hydrophobic and porous polystyrene-divinylbenzene-based resin, was used (10%wt.). The reaction medium was shaken at 300rpm and 30°C. Results are shown in Figure 7.

Figure 7 shows that *Pseudomonas sp.* lipase immobilized on a hydrophobic resin is also capable of catalyzing the esterification of free fatty acids to form fatty acid methyl esters and water by-product. The results show that the lipase preparation maintained its esterification/transesterification activity in a medium containing 1% sodium bicarbonate solution of 0.1M over more than 25 cycles using the same batch of biocatalyst without the observation of any significant loss of activity.

### Example 6

The transesterification of fish oil with ethanol after 6 hours of reaction using the same batch of biocatalyst in multiple batch experiments. *Reaction conditions:* Refined fish oil (20g) containing 1% sodium bicarbonate solution of 0.1M. Ethanol (2.5ml) was added stepwise in three equivalent batches each one hour apart. Lipases derived from *Thermomyces lanuginosa* and *Pseudomonas sp.* immobilized on Amberlite^{R} XAD 1600, were used separately (10%wt.). The reaction medium was shaken at 300rpm and 30°C. Results are shown in Figure 8.

Figure 8 shows that both lipases derived from *Thermomyces lanuginosa* and *Pseudomonas sp.* immobilized on hydrophobic resins are also capable of catalyzing the transesterification of fish oil triglycerides with ethanol to form fatty acid ethyl esters and glycerol by-product. The results also show that both biocatalyst preparations maintained their transesterification activity in the presence of 1% sodium bicarbonate solution without significant activity losses over more than 20 cycles using the same batch of biocatalyst.

### Example 7

The transesterification of Tallow fat with ethanol after 6 hours of reaction using the same batch of biocatalyst in multiple batch experiments.

*Reaction conditions:* Tallow fat (16g) containing fatty acid ethyl ester of tallow fat (4g) and 1% potassium carbonate solution of 1M. Ethanol (2.5ml) was added stepwise in three equivalent batches each one hour apart. Lipases derived from *Thermomyces lanuginose, Pseudomonas sp.* immobilized on Amberlite^{R} XAD 1600 (10%wt.) were used separately or in combination at an equivalent ratio. The reaction medium was shaken at 300rpm and 37°C. Results are shown in Figure 9.

Figure 9 shows that both lipases derived from *Thermomyces lanuginosa* and *Pseudomonas sp.* separately or in combination immobilized on hydrophobic resins are also capable of catalyzing the transesterification of tallow fat triglycerides with ethanol to form fatty acid ethyl esters and glycerol by-product. The feedstock of the reaction medium was comprised of tallow fat (80%) and fatty acid ethyl esters derived from tallow fat in order to lower the melting point of the reaction medium. The results presented in Figure 9 show that all biocatalysts retained more than 80% of their initial activity in the presence of mild alkaline solution, such as potassium carbonate of 1M, when the same batch of biocatalysts were used in 100 consecutive cycles.

### Example 8

The treatment of the transesterification/esterification reaction medium obtained after 4 hours containing FFA value of 7 mg KOH/1g using *Pseudomonas sp.* lipase or *Thermomyces lanuginosa* lipase immobilized on hydrophobic porous resins with *Candida Antarctica B* lipase immobilized on a hydrophobic porous resin and methanol (ratio of 1:10 on molar basic between FFA and methanol, respectively) using the same batch of biocatalyst (10%wt.) in multiple batch experiments. The reaction medium was shaken at 300rpm and 30°C. Results are shown in Figure 10.

Figure 10 shows that the transesterification reaction medium obtained after treatment either with *Thermomyces lanuginosa* lipase or *Pseudomonas sp.* lipase as described above, which typically contain FFAs values of 3-7 mg KOH/1g, can be treated with *Candida antarctica* B lipase immobilized on either hydrophilic or hydrophobic support, results in reducing the FFA value down to less than 2 mg KOH/1g. The immobilized lipase can maintain its activity in more than 100 cycles.

### Example 9

Transesterification/esterification of waste-cooking oil containing 10% **FFA** with methanol to form biodiesel, water and glycerol using the first embodiment of the system illustrated in Figure **11****.**

*Reaction conditions:* Waste-cooking oil (1100g) containing 2% of sodium bicarbonate solution of 0.1M and methanol (140g) were first premixed in pre-reaction preparation vessel **140** to form an emulsion, which was then introduced to the reactor vessel **120** having an internal volume **V2** of about 2 liters. The reaction mixture was mixed in the reactor vessel **120** with a lipase derived from *Thermomyces lanuginosa* immobilized on a hydrophobic and porous polystyrene-divinylbenzene-based resin (30% wt of the oil) for 6 hours at 30°C. The reaction mixture was filtered off through the filter **125** and fed to product separation vessel **160.** Glycerol and excess of water were removed from the reaction mixture in the product separation vessel **160.** The upper phase containing of the fatty acid methyl esters and the unreacted glycerides were re-introduced to the reactor vessel **120** via rerouting line 165, and stirring in the reactor vessel **120** was resumed after the addition of methanol (110g) in to the reaction medium in the reactor vessel **120.** The conversion to methyl ester after 2 hours was 98%. An emulsified reaction medium (prepared emulsion) containing waste-cooking oil (83% wt), methanol (15%) and sodium bicarbonate solution of 0.1M (2%) was continuously fed into the reactor vessel **120** at a flow rate of about 30ml/min. The conversion to fatty acid methyl esters was maintained to more than 3 months without significant activity losses when using the same batch of biocatalyst derived from *Thermomyces lanuginosa* lipase immobilized on a macroporous hydrophobic resin.

## Claims

1. A process selected from:
(A) a process for the transesterification/esterification of a fatty acid source with an alcohol, to form fatty acid alkyl esters, comprising reacting the said fatty acid source and an alcohol or an alcohol donor in the presence of an immobilized lipase preparation, wherein the immobilized lipase preparation comprises at least one lipase immobilized on a hydrophobic porous support and the reaction medium contains an aqueous alkaline buffer solution; and
(B) a process for the transesterification/esterification of a fatty acid source selected from triglycerides, diglycerides, monoglycerides and any mixture thereof, said mixture optionally further comprising free fatty acids, with an alcohol, to form fatty acid alkyl esters, comprising reacting the said fatty acid source and an alcohol in the presence of an immobilized lipase preparation, wherein the immobilized lipase preparation comprises at least one lipase immobilized on a hydrophobic porous support and wherein water is added to said fatty acid source or to the reaction medium.

2. The process of claim 1, which is process (A) and wherein the aqueous buffer solution has a pH from 7 to about 11.

3. The process of claim 1, which is process (B) and wherein the water is in the form of a water solution with a pH of from 3 to 11.

4. The process of any one of the preceding claims, wherein said alcohol is a short-chain alcohol; or wherein said alcohol donor is a mono-alkyl ester, such as methyl acetate or a di-alkyl carbonate, such as di-methyl carbonate, serving also as a source for mild alkaline reagent in the reaction medium.

5. The process of any one of the preceding claims, wherein said at least one lipase is a lipase derived from any one *Rhizomucor miehei, Pseudomonas sp., Rhizopus niveus, Mucor javanicus, Rhizopus oryzae, Aspergillus niger, Penicillium camembertii, Alcaligenes sp., Acromobacter sp., Burkholderia sp., Thermomyces lanuginosa, Chromobacterium viscosum, Candida antarctica B, Candida rugosa, Candida antarctica* A, papaya seeds and pancreatin.

6. The process of any one of the preceding claims, wherein said at least one immobilized lipase is capable of catalyzing the esterification of free fatty acids to yield fatty acid alkyl esters and water as by-product, and the transesterification of triglycerides and partial glycerides to yield fatty acid alkyl esters and glycerol as by-product.

7. The process of claim 1 or 2, wherein the process is process (A), or the process of any one of claims 3 to 6, wherein said lipase preparation comprises at least two lipases which may be each separately immobilized on a hydrophobic support or co-immobilized on the same hydrophobic support, and wherein said at least two lipases possess identical or different regio-specificity.

8. The process of any one of the preceding claims, wherein said support is any one of hydrophobic aliphatic polymer-based support and hydrophobic aromatic polymer-based support.

9. The process of any one of claims 1 to 7, wherein said support is porous or non-porous inorganic support, which may be hydrophobic or is coated with hydrophobic organic material.

10. The process of any one of the preceding claims wherein said fatty acid source is any one of plant oil, animal fat, algal oil, fish oil, waste oil, brown grease and any mixtures thereof.

11. The process of any one of the preceding claims , wherein said fatty acid source comprises free fatty acids, mono-, di- or tri-glycerides, their mixtures at any ratio, fatty acid esters and amides, in the absence or presence of other minor fatty acid derivatives such as phospholipids and sterol esters, said fatty acid source is unrefined, refined, bleached, deodorized or any of their combinations.

12. The process of any one of the preceding claims wherein said alcohol is methanol and said resulting fatty acid esters are fatty acid methyl esters (FAME - Biodiesel), or wherein said alcohol is a medium-chain fatty alcohol (C₆-C₁₀) or long-chain fatty alcohol (C₁₂-C₂₂).

13. The process of any one of the preceding claims, wherein said immobilized lipase is used in continuous stirred-tank reactors or in packed-bed column reactors operating in batch or continuous modes.

14. A system which is:
(A) a system for the transesterification/esterification of a fatty acid source with an alcohol, to form fatty acid alkyl esters, comprising:
a reaction vessel for reacting a reaction medium including the said fatty acid source and at least one of an alcohol and an alcohol donor in the presence of an immobilized lipase preparation, wherein the immobilized lipase preparation comprises at least one lipase immobilized on a hydrophobic porous support and the reaction medium contains an aqueous alkaline buffer solution; or
(B) a system for the transesterification/esterification of a fatty acid source selected from triglycerides, diglycerides, monoglycerides and any mixture thereof, said mixture optionally further comprising free fatty acids, with an alcohol, to form fatty acid alkyl esters, comprising:
a reaction vessel for reacting a reaction medium including the said fatty acid source and at least one of an alcohol and an alcohol donor in the presence of an immobilized lipase preparation, wherein the immobilized lipase preparation comprises at least one lipase immobilized on a hydrophobic porous support and wherein water is added to said fatty acid source or to the reaction medium.

15. The process of any one of claims 1 to 13, conducted in a system as defined in claim 14.

## Patentansprüche

1. Verfahren, ausgewählt aus:
(A) einem Verfahren für die Umesterung/Veresterung einer Fettsäurequelle mit einem Alkohol, um Fettsäurealkylester zu bilden, umfassend die Umsetzung der Fettsäurequelle und eines Alkohols oder eines Alkoholdonors in Gegenwart eines immobilisierten Lipasepräparats, wobei das immobilisierte Lipasepräparat mindestens eine Lipase, immobilisiert auf einem hydrophoben porösen Träger, umfasst und das Reaktionsmedium eine wässrige alkalische Pufferlösung enthält; und
(B) einem Verfahren für die Umesterung/Veresterung einer Fettsäurequelle, ausgewählt aus Triglyceriden, Diglyceriden, Monoglyceriden und jeglichem Gemisch davon, wobei das Gemisch gegebenenfalls des Weiteren freie Fettsäuren umfasst, mit einem Alkohol, um Fettsäurealkylester zu bilden, umfassend die Umsetzung der Fettsäurequelle und eines Alkohols in Gegenwart eines immobilisierten Lipasepräparats, wobei das immobilisierte Lipasepräparat mindestens eine Lipase, immobilisiert auf einem hydrophoben porösen Träger, umfasst und wobei Wasser zu der Fettsäurequelle oder zu dem Reaktionsmedium gegeben wird.

2. Verfahren nach Anspruch 1, welches Verfahren (A) ist und wobei die wässrige Pufferlösung einen pH-Wert von 7 bis etwa 11 hat.

3. Verfahren nach Anspruch 1, welches Verfahren (B) ist und wobei das Wasser in Form einer Wasserlösung mit einem pH-Wert von 3 bis 11 ist.

4. Verfahren nach einem der vorstehenden Ansprüche, wobei der Alkohol ein kurzkettiger Alkohol ist oder wobei der Alkoholdonor ein Monoalkylester, wie Methylacetat, oder ein Dialkylcarbonat, wie Dimethylcarbonat, ist, der auch als Quelle für ein mildes alkalisches Reagens in dem Reaktionsmedium dient.

5. Verfahren nach einem der vorstehenden Ansprüche, wobei die mindestens eine Lipase eine Lipase ist, abgeleitet von einem von *Rhizomucor miehei, Pseudomonas sp*., *Rhizopus niveus, Mucor javanicus, Rhizopus oryzae, Aspergillus niger, Penicillium camembertii, Alcaligenes sp., Acromobacter sp., Burkholderia sp., Thermomyces lanuginosa, Chromobacterium viscosum, Candida antarctica B, Candida rugosa, Candida antarctica A*, Papayasamen und Pancreatin.

6. Verfahren nach einem der vorstehenden Ansprüche, wobei die mindestens eine immobilisierte Lipase geeignet ist zur Katalyse der Veresterung von freien Fettsäuren, um Fettsäurealkylester und Wasser als Nebenprodukt zu erhalten und zur Umesterung von Triglyceriden und Teilglyceriden, um Fettsäurealkylester und Glycerin als Nebenprodukt zu erhalten.

7. Verfahren nach Anspruch 1 oder 2, wobei das Verfahren Verfahren (A) ist, oder das Verfahren nach einem der Ansprüche 3 bis 6, wobei das Lipasepräparat mindestens zwei Lipasen, die jeweils separat immobilisiert auf einem hydrophoben Träger oder co-immobilisiert auf dem gleichen hydrophoben Träger sein können, umfasst, und wobei die mindestens zwei Lipasen identische oder unterschiedliche Regiospezifizität besitzen.

8. Verfahren nach einem der vorstehenden Ansprüche, wobei der Träger einer von hydrophobem aliphatischem Polymer-basiertem Träger und hydrophobem aromatischem Polymer-basiertem Träger ist.

9. Verfahren nach einem der Ansprüche 1 bis 7, wobei der Träger ein poröser oder nichtporöser anorganischer Träger ist, der hydrophob sein kann oder beschichtet ist mit einem hydrophoben organischen Material.

10. Verfahren nach einem der vorstehenden Ansprüche, wobei die Fettsäurequelle eine aus Pflanzenöl, tierischem Fett, Algenöl, Fischöl, Abfallöl, Braunfett und jeglichen Gemischen davon ist.

11. Verfahren nach einem der vorstehenden Ansprüche, wobei die Fettsäurequelle freie Fettsäuren, Mono-, Di- oder Triglyceride, deren Gemische in jeglichem Verhältnis, Fettsäureester und Amide, in Abwesenheit oder Anwesenheit von anderen geringfügigen Fettsäurederivaten, wie Phospholipiden und Sterolestern, umfasst, wobei die Fettsäurequelle unraffiniert, raffiniert, gebleicht, desodoriert oder eine ihrer Kombinationen ist.

12. Verfahren nach einem der vorstehenden Ansprüche, wobei der Alkohol Methanol ist und die resultierenden Fettsäureester Fettsäuremethylester (FAME-Biodiesel) sind,
oder
wobei der Alkohol ein mittelkettiger Fettalkohol (C₆-C₁₀) oder langkettiger Fettalkohol (C₁₂-C₂₂) ist.

13. Verfahren nach einem der vorstehenden Ansprüche, wobei die immobilisierte Lipase in kontinuierlich gerührten Tankreaktoren oder in Festbett-Kolonnenreaktoren, die in diskontinuierlicher oder kontinuierlicher Weise arbeiten, verwendet wird.

14. System, welches:
(A) ein System ist für die Umesterung/Veresterung einer Fettsäurequelle mit einem Alkohol, um Fettsäurealkylester zu bilden, umfassend:
ein Reaktionsgefäß für die Umsetzung eines Reaktionsmediums, das die Fettsäurequelle und mindestens einen Alkohol und einen Alkoholdonor in Gegenwart eines immobilisierten Lipasepräparats beinhaltet, wobei das immobilisierte Lipasepräparat mindestens eine Lipase, immobilisiert auf einem hydrophoben porösen Träger, umfasst und das Reaktionsmedium eine wässrige alkalische Pufferlösung enthält; oder
(B) ein System ist für die Umesterung/Veresterung einer Fettsäurequelle, ausgewählt aus Triglyceriden, Diglyceriden, Monoglyceriden und jeglichem Gemisch davon, wobei das Gemisch gegebenenfalls des Weiteren freie Fettsäuren umfasst, mit einem Alkohol, um Fettsäurealkylester zu bilden, umfassend:
ein Reaktionsgefäß für die Umsetzung eines Reaktionsmediums, das die Fettsäurequelle und mindestens einen Alkohol und einen Alkoholdonor in Gegenwart eines immobilisierten Lipasepräparats beinhaltet, wobei das immobilisierte Lipasepräparat mindestens eine Lipase, immobilisiert auf einem hydrophoben porösen Träger, umfasst und wobei Wasser zu der Fettsäurequelle oder zu dem Reaktionsmedium gegeben wird.

15. Verfahren nach einem der Ansprüche 1 bis 13, durchgeführt in einem System wie in Anspruch 14 definiert.

## Revendications

1. Procédé choisi parmi :
A) un procédé de trans-estérification ou d'estérification d'une source d'acides gras, à l'aide d'un alcool, pour former des esters alkyliques d'acides gras, lequel procédé comporte le fait de faire réagir ladite source d'acides gras et un alcool ou un donneur d'alcool en présence d'une préparation de lipase immobilisée, et dans lequel procédé la préparation de lipase immobilisée comprend au moins une lipase immobilisée sur un support poreux hydrophobe, et le milieu réactionnel contient une solution tampon aqueuse alcaline ;
B) et un procédé de trans-estérification ou d'estérification d'une source d'acides gras, choisie parmi les triglycérides, diglycérides et monoglycérides et tout mélange de tels corps, lequel mélange, en option, contient en outre des acides gras libres, à l'aide d'un alcool, pour former des esters alkyliques d'acides gras, lequel procédé comporte le fait de faire réagir ladite source d'acides gras et un alcool en présence d'une préparation de lipase immobilisée, et dans lequel procédé la préparation de lipase immobilisée comprend au moins une lipase immobilisée sur un support poreux hydrophobe, et de l'eau est ajoutée à ladite source d'acides gras ou au milieu réactionnel.

2. Procédé conforme à la revendication 1, qui est un procédé (A) et dans lequel la solution tampon aqueuse présente un pH valant de 7 à environ 11.

3. Procédé conforme à la revendication 1, qui est un procédé (B) et dans lequel l'eau se présente sous la forme d'une solution aqueuse qui présente un pH valant de 3 à 11.

4. Procédé conforme à l'une des revendications précédentes, dans lequel ledit alcool est un alcool à chaîne courte ou dans lequel ledit donneur d'alcool est un ester monoalkylique, tel de l'acétate de méthyle, ou un carbonate dialkylique, tel du carbonate de diméthyle, qui sert également de source de réactif alcalin modéré dans le milieu réactionnel.

5. Procédé conforme à l'une des revendications précédentes, dans lequel ladite lipase au nombre d'au moins une est une lipase qui provient de l'une des sources suivantes : *Rhizomucor miehei, Pseudomonas sp., Rhizopus nivei, Mucor javanicus, Rhizopus oryzae, Aspergillus niger, Penicillium camembertii, Alcaligenes sp., Acromobacter sp., Burkholderia sp., Thermomyces lanuginosa, Chromobacterium viscosum, Candida antarctica B, Candida rugosa, Candida antarctica A,* graines de papaye et pancréatine.

6. Procédé conforme à l'une des revendications précédentes, dans lequel ladite lipase immobilisée au nombre d'au moins une est capable de catalyser l'estérification des acides gras libres donnant des esters alkyliques d'acides gras, et de l'eau en tant que sous-produit, ainsi que la trans-estérification des triglycérides et glycérides partiels donnant des esters alkyliques d'acides gras, et du glycérol en tant que sous-produit.

7. Procédé conforme à la revendication 1 ou 2, ledit procédé étant un procédé (A), ou procédé conforme à l'une des revendications 3 à 6, dans lequel ladite préparation de lipase comprend au moins deux lipases, qui peuvent être immobilisées chacune séparément sur un support hydrophobe ou immobilisées conjointement sur le même support hydrophobe, et dans lequel ces lipases au nombre d'au moins deux possèdent des régiospécificités identiques ou différentes.

8. Procédé conforme à l'une des revendications précédentes, dans lequel ledit support est n'importe quel support à base de polymère aliphatique hydrophobe ou à base de polymère aromatique hydrophobe.

9. Procédé conforme à l'une des revendications 1 à 7, dans lequel ledit support est un support inorganique poreux ou non-poreux, qui peut être hydrophobe ou être revêtu d'un matériau organique hydrophobe.

10. Procédé conforme à l'une des revendications précédentes, dans lequel ladite source d'acides gras est l'une des suivantes : une huile végétale, une graisse animale, une huile d'algue, une huile de poisson, une huile résiduaire, une graisse résiduaire dite "graisse brune", et tout mélange de telles huiles et/ou graisses.

11. Procédé conforme à l'une des revendications précédentes, dans lequel ladite source d'acides gras comprend des acides gras libres, des monoglycérides, diglycérides ou triglycérides, des mélanges de ceux-ci en toutes proportions, des esters et amides d'acides gras, en l'absence ou en présence d'autres dérivés mineurs d'acides gras, tels des phospholipides ou des esters de stérol, et ladite source d'acides gras se présente à l'état non-purifié, purifié, blanchi ou désodorisé ou dans n'importe quelle combinaison de ces états.

12. Procédé conforme à l'une des revendications précédentes, dans lequel ledit alcool est du méthanol et lesdits esters d'acides gras résultants sont des esters méthyliques d'acides gras (Biodiesel EMAG), ou dans lequel ledit alcool est un alcool gras à chaîne moyenne (en C₆ à C₁₀) ou un alcool gras à chaîne longue (en C₁₂ à C₂₂).

13. Procédé conforme à l'une des revendications précédentes, dans lequel ladite lipase immobilisée est utilisée dans des réacteurs à cuve sous agitation fonctionnant en mode continu ou dans des réacteurs en colonne à lit de garnissage fonctionnant en mode continu ou discontinu.

14. Système qui est :
A) un système servant à la trans-estérification ou l'estérification d'une source d'acides gras, à l'aide d'un alcool, pour former des esters alkyliques d'acides gras, lequel système comporte un récipient de réaction servant à faire réagir un milieu réactionnel, comprenant ladite source d'acides gras et au moins l'un d'un alcool et d'un donneur d'alcool, en présence d'une préparation de lipase immobilisée, et dans lequel système la préparation de lipase immobilisée comprend au moins une lipase immobilisée sur un support poreux hydrophobe, et le milieu réactionnel contient une solution tampon aqueuse alcaline ;
B) ou un système de trans-estérification ou d'estérification d'une source d'acides gras, choisie parmi les triglycérides, diglycérides et monoglycérides et tout mélange de tels corps, lequel mélange, en option, contient en outre des acides gras libres, à l'aide d'un alcool, pour former des esters alkyliques d'acides gras, lequel système comporte un récipient de réaction servant à faire réagir un milieu réactionnel, comprenant ladite source d'acides gras et au moins l'un d'un alcool et d'un donneur d'alcool, en présence d'une préparation de lipase immobilisée, et dans lequel système la préparation de lipase immobilisée comprend au moins une lipase immobilisée sur un support poreux hydrophobe, et de l'eau est ajoutée à ladite source d'acides gras ou au milieu réactionnel.

15. Procédé conforme à l'une des revendications 1 à 13, mis en oeuvre dans un système conforme à la revendication 14.
